# EUROPEAN PATENT SPECIFICATION

(11) **EP 2 156 840 B1**
(45) Date of publication and mention of the grant of the patent: **10.08.2011**
(21) Application number: 09007995.5
(22) Date of filing: 26.01.1995
(51) Int. Cl.: A61K 31/58, A61P 11/02, A61P 27/14, A61K 9/00

(54) **Use of mometasone furoate for treating airway passage and lung diseases**
Verwendung von Mometason-Furoat zur Behandlung von Luftweg- und Lungenerkrankungen
Utilisation du furoate de mometasone pour le traitement des affections pulmonaires et des voies respiratoires

(30) Priority: 27.01.1994 US 188372
(43) Date of publication of application: 24.02.2010
(62) Divisional of application: 01119613.6
(73) Proprietor: Schering Corporation, Kenilworth, NJ 07033-0530 (US)
(72) Inventor: Sequeira, Joel A., Ocean, NJ 07712 (US); Cuss, Francis M., Basking Ridge, New Jersey 07920 (US); Nolop, Keith B., Milburn, New Jersey 07041 (US); Chaudry, Imtiaz A., North Caldwell, NY 07006 (US); Nagabhushan, Nagamani, Parsippany, New Jersey (US); Patrick, James E., Belle Meade, New Jersey 08502 (US); Cayen, Mitchell, Bedminister New Jersey 07921 (US)
(74) Representative: Hussain, Deeba

(56) References cited:
- EP-A- 0 518 600
- EP-A- 0 518 601
- C.J.WANG ET AL.: "A Sensitive Enzyme Immunoassay (EIA) for Quantitation of Mometasone Furoate (SCH 32088) Directly in Biological Fluids" FASEB J., vol. 6, no. 4, 1992, page A1302, XP002183700
- H.J.LEE ET AL.: "ANTI-INFLAMMATORY STEROIDS: RESEARCH TRENDS AND NEW COMPOUNDS" DRUGS TODAY, vol. 25, no. 9, September 1989 (1989-09), pages 577-588, XP002183702
- WANG C-J ET AL: "A competitive enzyme immunoassay for the direct determination of mometasone furoate (SCH 32088) in human plasma" JOURNAL OF PHARMACEUTICAL AND BIOMEDICAL ANALYSIS, NEW YORK, NY, US LNKD- DOI:10.1016/0731-7085(92)80067-W, vol. 10, no. 7, 1 January 1992 (1992-01-01), pages 473-479, XP002249818 ISSN: 0731-7085

## Description

### INTRODUCTION TO THE INVENTION

This invention relates to the use of an aqueous suspension mometasone furoate for the preparation of medicaments for intranasal once-a-day treating of a corticosteroid-responsive disease of the upper and lower airway passages and lungs, which is allergic rhinitis in the substantial absence of absorption systemically into the bloodstream of the mometasone furoate and the side effects associated with such systemic absorption.

Mometasone furoate is a corticosteroid approved for topical dermatologic use to treat inflammatory and/or pruritic manifestations of corticosteroid-responsive dermatoses. The compound may be prepared in accordance with the procedures disclosed in U.S. Patents 4,472,393, 4,731,447, and 4,873,335.

Certain corticosteroids, e.g., beclomethasone dipropionate are commercially available for the treatment of diseases of airway passages and lungs such as rhinitis and bronchial asthma. However, the art teaches that not every corticosteroid having topical anti-inflammatory activity is active in treating rhinitis and/or asthma. Furthermore, even though a topically active corticosteroid may exhibit activity in treating bronchial asthma, the long term use of such steroids has been limited by the occurrence of serious systemic side-effects, including hypothalamic-pituitary-adrenal (HPA) axis suppression. The introduction of topically active steriods administered by metered-dose inhalation has greatly reduced but not eliminated the detrimental system side-effects of steroid therapy in the treatment of asthma. Unfortunately, however, a large portion of an inhaled corticosteriod dose is swallowed by the patient. Since certain corticosteroids are readily bioavailable, the swallowed portion of the dose may reach the systemic circulation through the gastro-intestinal tract and may cause unwanted systemic side-effects. Some corticosteroids currently approved for treating asthma have systemic bioavailability after oral ingestion of greater than 10% (budesonide) or even 20% (triamcinolone acetonide and flunisolide) of the inhalation dose. Thus, a topically active steroid which is not readily bioavailable would provide a therapeutic advantage over other topically active corticosteroids that are more systematically bioavailable and it would also be superior to any corticosteroid orally administered by the oral swallowing of, for example, a solution, tablet or capsule.

EP-A-0518601 and EP-A-0518600 both relate to non-chlorofluorocarbonaerosol formulations containing a medicament which may be mometasone fuorate and the use of the formulations for treating asthma.

Wang et al., "A Sensitive Enzyme Immunoassay (EIA) for Quantitation of Mometasone Furoate (SCH 32088) Directly in Biological Fluids", Faseb J., Vol. 6, No. 4, 1992, page A1302, XP002183700 - Abstract" discuss a competative enzyme immunoassay for the direct determination of mometasone furoate in human plasma. The formulation used is a solution of mometasone furoate administered by oral swallowing to the patient.

Lee et al., "Anti-Inflammatory Steroids: Research Trends and New Compounds", Drugs today, Vol. 25, No. 9, September 1989 (1989-09), pages 577-588, XP002183702, page 580, left-hand column" identifies large groups of anti-inflammatory steroids including mometasone furoate.

Discovering an effective corticosteroid for treating diseases such as asthma with low systemic side-effects is unpredictable. For example, the corticosteroid tipredane exhibited not only good initial anti-inflammatory activity against asthma but also low systemic side effects. However, development of tipredane for treating asthma has been discontinued because clinical trials have not demonstrated a level of efficacy in treating asthma which would be considered therapeutically useful. It has recently been disclosed that butixocort propionate, another potent topical anti-inflammatory corticosteroid having reportedly low systemic side-effects is under development (Phase II) for treating chronic bronchial asthma. While the clinical results currently available from the Phase II studies show butixocort propionate has some efficacy, it remains to be seen if the efficacy in treating asthma will be sufficient to justify continuing the clinical development.

Thus, it would be desirable to find a corticosteroid which is therapeutically effective in treating disease of the airway passages and lungs such as asthma and which also exhibits low bioavailability and low systemic side-effects when it is administered intra-nasally or by oral inhalation.

### SUMMARY OF THE INVENTION

The present invention provides an aqueous suspension of mometasone furoate for use as a medicament for the effective intranasal treatment of allergic rhinitis defined in claim 1 and dependent claims 2 to 5.

### BRIEF DESCRIPTION OF THE FIGURES

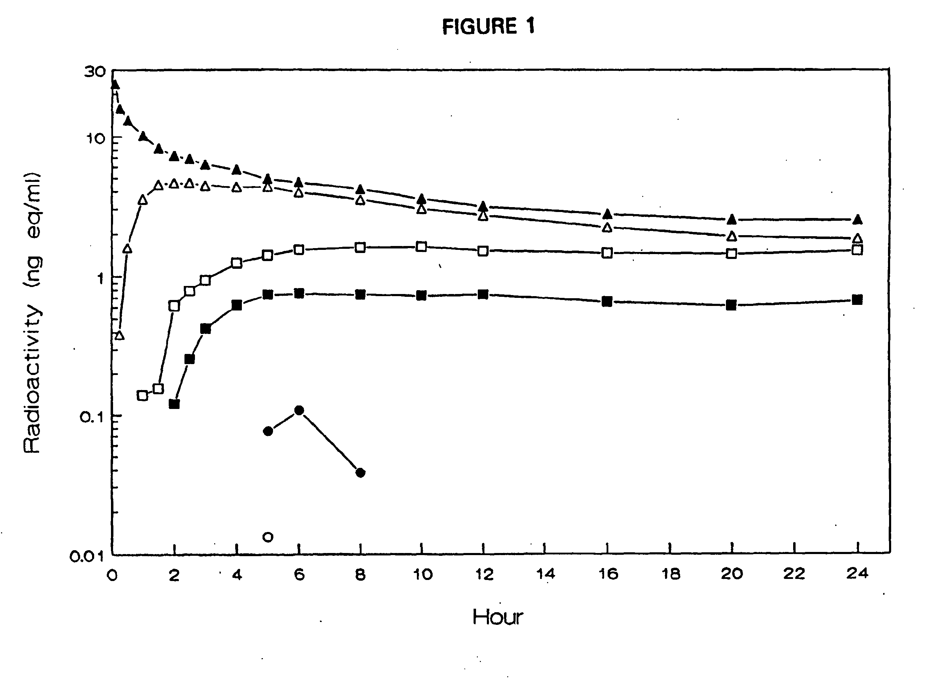

Figure 1 graphically illustrates the variation with time (measured in hours) of the plasma concentrations of total radioactivity (measured in ng-eq/mL) following administration of tritium-labelled mometasone furoate by various formulations and routes of administration to male volunteers. The curve plotted with the darkened circles (•) represents the variations of plasma concentrations with time after administration of radio-labelled drug by oral suspension; the curve plotted with open circles (o) represents the variation of plasma concentrations with time after administration of drug by nasal spray; the curve plotted with the darkened squares (■) represents the variation of plasma concentrations with time after administration by a metered dose inhaler; the curve plotted with the open squares (□) represent the variation of plasma concentrations with time after administration of drug by Gentlehaler; the curve plotted with the darkened triangles (Δ) represents the variation of plasma concentrations with time after administration of drug by the intravenous route and the curve plotted with the open triangles (Δ) represent the variations of plasma concentration with time after administration of the radio-labelled drug via oral solution. See Tables in Results section hereinafter.

### DETAILED DESCRIPTION OF THE INVENTION AND

### OF THE PREFERRED EMBODIMENTS

Although corticosteroids have been effective in treating airway passage diseases such as asthma, such treating with corticosteroids may often cause systemic side-effects such as suppression of hypothalamic-pituitary-adrenocortical ("HPA") axis function by reducing corticotrophin (ACTH) production, which in turn leads to a reduced cortisol secretion by the adrenal gland.

We have surprisingly discovered that mometasone furoate exhibits superior anti-inflammatory effects in treating airway passage diseases such as allergic rhinitis by acting on surfaces of the upper and lower airways passages and lungs while having a substantially minimum systemic effect. The substantial minimization of the systemic effect of mometasone furoate administered intranasally or by oral inhalation has been measured by High Performance Liquid Chromatography (HPLC) metabolite profiling of plasma radioactivity of mometasone furoate, its substantially complete (>98%) first-pass metabolism in the liver and by a minimal reduction in cortisol secretion levels.

When mometasone furoate is administered by nasal inhalation, there is substantial absence of absorption systemically into the bloodstream of mometasone furoate i.e. there is essentially no parent drug (substantially less than 1% of mometasone furoate) which reaches the bloodstream from the gastro-intestinal tract. Any mometasone furoate found in the bloodstream after it has been administered by nasal inhalation has already passed through the lungs and/or airway passage tissue. Therefore, there is no "wasted" drug (i.e., drug that reaches the relevant tissue in the lungs and/or airways only via the bloodstream). Thus, mometasone furoate is an ideal drug for treating diseases of the airway passages and lungs such as asthma and allergic rhinitis.

In addition to the superb safety profile exhibited by mometasone furoate administered to patients with allergic rhinitis in accordance with the present invention, mometasone furoate also exhibits an unexpected higher level of efficacy in treating allergic rhinitis than the superb safety profile would suggest.

The term "corticosteroid-responsive disease of the airway passage ways and lungs" as used herein means those allergic diseases of the upper or lower airway passages or of the lungs which are treatable by administering corticosteroids such as mometasone furoate. Typical corticosteroid-responsive diseases include asthma, allergic and non-allergic rhinitis as well as non-malignant proliferative and inflammatory diseases of the airways passages and lungs.

The methods of this invention are useful in treating allergic rhinitis .

The term "allergic rhinitis" as used herein means any allergic reaction of the nasal mucosa and includes hay fever (seasonal allergic rhinitis) and perennial rhinitis (non-seasonal allergic rhinitis) which are characterized by seasonal or perennial sneezing, rhinorrhea, nasal congestion, pruritis and eye itching, redness and tearing.

The mometasone furoate administered intranasally to treat disease of the lower and/or upper airway passages and/or lungs may be used as monotherapy or as adjuvant therapy with for example cromolyn sodium or nedocromil sodium (available from Fisons); immunosuppressive agents such as methotrexate sodium (available from Astra Pharmaceutical Products, Inc.), oral gold, or cyclosporine A (available from Sandoz under the SANDIMMUNE^{®} tradename); bronchodilators such as albuterol (available from Schering Corporation under the PROVENTIL^{®} tradename) or theophylline (available from Key Pharmaceuticals of Schering Corporation under the Theo-Dur^{®} tradename).

Mometasone furoate may be also administered in specific, measured amounts in the form of an aqueous suspension by use of a pump spray bottle such as the bottles used to deliver VANCENASE AQ^{®} Nasal Spray as well as the spray bottle disclosed in the Schering Corporation Industrial Design Deposit DM/026304, registered by the Hague Union on June 1, 1993 (each are available from Schering Corporation). The aqueous suspension compositions of the present invention may be prepared by admixing mometasone furoate or mometasone furoate monohydrate (preferably mometasone furoate monohydrate) with water and other pharmaceutically acceptable excipients. See International Application No. PCT/US91/06249 especially Examples 1-5 for preparation of mometasone furoate monohydrate and aqueous suspensions containing same. The aqueous suspensions of the invention may contain from about 0.01 to 10.0 mg, preferably 0.1 to 10.0 mg of mometasone furoate monohydrate per gram of suspension. The aqueous suspension compositions according to the present invention may contain, inter alia, water, auxiliaries and/or one or more of the excipients, such as: suspending agents, e.g., microcrystalline cellulose, sodium carboxymethylcellulose, hydroxpropyl-methyl cellulose; humectants, e.g. glycerin and propylene glycol; acids, bases or buffer substances for adjusting the pH, e.g., citric acid, sodium citrate, phosphoric acid, sodium phospate as well as mixtures of citrate and phosphate buffers; surfactants, e.g. Polysorbate 80; and antimicrobial preservatives, e.g., benzalkonium chloride, phenylethyl alcohol and potassium sorbate.

Based on the judgment of the attending clinician, the amount of mometasone furoate administered and the treatment regimen used will, of course, be dependent on the age, sex and medical history of the patient being treated, the severity of the specific asthmatic or non-malignant pulmonary disease condition and the tolerance of patient to the treatment regimen as evidenced by local toxicity (e.g., nasal irritation and/or bleeding) and by systemic side-effects (e.g. cortisol level). Cortisol (also referred to as hydrocortisone) is the major natural glucocorticosteroid elaborated by the adrenal cortex.

For the treatment of allergic rhinitis , the substantially non-systematically bioavailable amount of mometasone furoate which may be administered as an aqueous suspension is in the range of 200 to 400 micrograms ("mcg") in single or divided doses.

In treating allergic rhinitis, the aqueous suspension of mometasone furoate may be administered intranasally by inserting an appropriate device (such as the pump spray bottle used to deliver Vancenase AQ^{®} Nasal Spray as well as the spray bottle disclosed in the Schering Corporation Industrial Design Deposit DM/026304 registered June 1, 1993) into each nostril. Active drug is then expelled (nasal spray device) or could be nasally inhaled (sniffed) as a powder. Efficacy is generally assessed in a double blind fashion by a reduction in nasal symptoms (e.g., sneezing, itching, congestion, and discharge). Other objective measurements (e.g., nasal peak flow and resistance) can be used as supportive indices of efficacy.

For treatment of allergic diseases of the lower airways and lung parenchyma the following dosage ranges of mometasone furoate may be used: for aqueous suspension for inhalation, the preferral dosage ranged from 25 to 800 mcg/100µ and the dosages are 25, 50, 100, 125, 150, 175, 200, 225, 250, 300, 400, 500 and 800 mcg/100µ of mometasone furoate in single or divided doses. The aqueous suspension of mometasone furoate has been found to be safe and effective in treating allergic rhinitis e.g. seasonal allergic rhinitis from 25 micrograms up to 1600 micrograms administered once-α-day; the preferred dosage range is 25-800 micrograms a day, although no improvement in treatment is topically found above 400 micrograms a day. The most preferred dosages are 25, 50 and 100 micrograms administered twice to each nostril, once-a-day for a total once-a-day dose of 100, 200 and 400 mcg. Typically 2-4 mL of the aqueous suspension of mometasone furoate monohydrate may be placed in a plastic nebulizer container and the patient would inhale for 2-10 minutes. The total dosage placed in such a container would be in the range of 300-3000 mcg. For any route of administration, divided or single doses may be used. For example, when a metered dose inhaler is used to deliver, for example, 500 mcg of aerosolized mometasone furoate, once-α-day, two puffs of 250 mcg would normally be used to deliver the aerosolized drug. When a plastic nebulizer container is used to deliver for example 200 micrograms a day of an aqueous suspension of mometasone furoate, two squeezes of 50 micrograms into each nostril would normally be used to deliver the drug. When the metered dose inhaler is used to deliver for example 200 mcg of anhydrous mometasone furoate, two puffs of 500 micrograms of an admixture of 100 mcg of mometasone furoate and 400 mcg of lactose once-α-day would normally be used to deliver the aerosolized drug.

The effectiveness of the methods of this invention can also be shown clinically in mammals, e.g. humans being afflicted with or susceptible to a non-malignant proliferative and/or inflammatory disease such as idophathic pulmonary fibrosis or using patients with inter alia the following entry criteria: 1. an improved Karnofsky performance status; (2) adequate pulmonary function for undergoing the required inhalation treatment satisfactorily as evidenced by (a) an improved forced expiratory volume (FEV) and (b) an improved forced vital capacity (FVC) and (3) no serious systemic infections and/or fever.

Similar results to those achieved in treating asthma are expected.

### RESULTS

Mometasone furoate (intranasally in the form of an aqueous suspension of mometasone furoate monohydrate) has been used for treating patients with seasonal allergic rhinitis. The term "seasonal allergic rhinitis" as used herein means a hypersensitivity response to seasonal pollens characterized by inflammation of the nasal mucous membranes, nasal discharge, sneezing and congestion.

Several Phase I studies have been completed using the aqueous nasal spray suspension formulation of mometasone furoate monohydrate. In a randomized, third party-blinded, placebo-controlled rising single-dose safety and tolerance study, the aqueous nasal spray suspension formulation was administered to eight healthy male volunteers. Doses were administered at 11 pm, and plasma cortisol concentrations were measured during the following 24-hour period. Compared to placebo, mometasbne furoate at doses of 1000 mcg, 2000 mcg, and 4000 mcg did not significantly affect the 24-hour area under the curve plasma cortisol profile (AUC 0-24).

In a follow-up multiple dose study, 48 normal male volunteers were empaneled in a randomized, third party-blinded, placebo and active-controlled parallel group study. Twelve volunteers in each of four groups received one of the following treatments for 28 days: A) Intranasal aqueous nasal spray suspension formulation of mometasone furoate monohydrate, 400 mcg/day; B) Intranasal aqueous nasal spray suspension formulation of mometasone furoate monohydrate, 1600 mcg/day; C) Intranasal placebo; D) Oral prednisone, 10 mg/day. All treatments were administered as once daily dosing in the morning. The mometasone furoate aqueous nasal spray formulation was well tolerated, and all patients completed the study. Neither of the 2 doses of the mometasone furoate aqueous nasal spray formulation were associated with any changes in cortisol secretion compared to placebo.

In addition, a single-dose absorption, excretion and metabolism study using 200 mcg of ³H-mometasone furoate as the nasal spray formulation was conducted in 6 normal male volunteers. When systemic absorption (based on urinary excretion) was compared to an intravenously administered dose of ³H-mometasone furoate, it was 8%. The plasma concentrations of parent drug could not be determined by metabolite profiling because the levels of plasma radioactivity were below the limit of quantification. These data are consistent with substantially less than 1 % of bioavailability of mometasone furoate. See Tables 1 to 2 herein below.

In a dose ranging safety and efficacy study, the mometasone furoate aqueous nasal spray formulation at doses of 50 mcg/day, 100 mcg/day, 200 mcg/day, 800 mcg/day or placebo was administered to 480 patients with seasonal allergic rhinitis for 4 weeks. All treatments were well tolerated; results of statistical analysis indicated that all doses of mometasone furoate were effective relative to placebo. These results showed that administration of an aqueous suspension of mometasone furoate as a nasal spray to patients with seasonal allergic rhinitis was effacious, well tolerated with little potential for systemic side effects and are consistent with the low oral bioavailability of mometasone furoate.

The term "rapid onset of action in treating allergic or seasonal allergic rhinitis" as used herein means that there is a clinically and statistically significant reduction in the total nasal symptom score from baseline for seasonal allergic rhinitis patients treated with mometasone furoate nasal spray with medium onset to moderate or complete relief at about 3 days (35.9 hours) compared to 72 hours for the patients treated with a placebo nasal spray. These results were obtained in a randomized, double-blind, multicenter, placebo-controlled, parallel group study to characterize the period between initiation of dosing with mometasone furoate nasal spray and onset of clinical efficacy as measured by the total nasal symptom score in symptomatic patients with seasonal allergic rhinitis. The study lasted 14 days in length. Data from 201 patients were used for analysis.

### A. Clinical Evaluations

### 1. Seasonal Allergy Rhinitis

a. Signs and symptoms were individually scored by the patient on the diary card, and by the investigator or designee at Screening and Baseline (Day 1), Day 4, Day 8, and Day 15 after treatment.

| Signs and Symptoms of Rhinitis | |
|---|---|
| Nasal | Non-Nasal |
| Nasal stuffiness/congestion | Itching/buring eyes |
| Rhinorrhea (nasal discharge/ | Tearing/watering eyes |
| runny nose) | Redness of eyes |
| Nasal itching, | Itching of ears or palate |
| Sneezing | |

All symptoms (nasal and non-nasal) were rated by the investigator or designee according to the following scale:

| | |
|---|---|
| 0 = None: | No signs/symptoms are evident |
| 1 = Mild: | Signs/symptoms are clearly present but minimal awareness; easily tolerated |
| 2 = Moderate: | Definite awareness of signs/symptoms which are bothersome but tolerable |
| 3 = Severe: | Signs/symptoms are hard to tolerate; may cause interference with activities of daily living and/or sleeping |

### 2. Overall Condition of Seasonal Allergic Rhinitis

The overall condition of rhinitis was evaluated by the investigator or designee and patient at the same time as symptoms, and scored according to the following criteria:

| | |
|---|---|
| 0 = None: | No signs/symptoms are evident |
| 1 = Mild: | Signs/symptoms are clearly present but minimal awareness; easily tolerated |
| 2 = Moderate: | Definite awareness of signs/symptoms which are bothersome but tolerable |
| 3 = Severe: | Signs/symptoms are hard to tolerate; may cause interference with activities of daily living and/or sleeping. |

In order to qualify for randomization, a patient must have had:
1. Nasal congestion ≥ 2 (moderate) at both Screening and Basline.
2. Total score of the four nasal symptoms ≥7 at both Screening and Baseline.
3. Overall condition ≥ 2 (moderate) at both Screening and Basline.

At visits after Basline, evaluations included the entire time period since the last visit, up to and including the time of the current observations.

3. Drug - Each patient was given a metered nasal pump spray bottle containing either an aqueous suspension of mometasone furoate or placebo. Dosing instructions on the bottle informed patient to deliver 2 sprays of drug (mometasone furoate 50 mcg/spray) or placebo into each nostril once-α-day, each morning.

### 4. Clinical Efficacy

### 1. Parameters

After the Baseline visit, each patient was instructed to enter into his/her diary the information about the time of onset of nasal relief and level of nasal symptom relief as no relief, slight, moderate, marked, or complete.

At Baseline and each follow-up visit, the physician evaluated the following signs and symptoms of allergic rhinitis, scored as 0=none, 1 =mild, 2=moderate, 3=severe.

### a. NASAL SYMPTOMS

nasal discharge
congestion/stuffiness
sneezing
itching

### b. TOTAL NASAL SCORE: sum of the 4 individual nasal scores

### c. COMPOSITE TOTAL SCORE: sum of the 8 nasal and non-nasal scores

The overall condition of rhinitis was also evaluated by both the physician and patient using the same scoring system.

At each follow-up visit post Baseline, the physician and patient evaluated the therapeutic response as 5=no relief, 4=slight relief, 3=moderate relief, 2=marked relief, 1=complete relief,

After the Basline visit, each morning and evening the patient completed a diary to assess the 8 signs and symptoms of allergic rhinitis as described above.

### RESULTS

The primary efficacy results are based on a survival analysis of the onset times of relief (defined as the first time patient experienced at least moderate relief of nasal symptoms) for the mometasone furoate nasal spray and placebo groups. In this analysis, patients reporting slight or no relief for the first 3 days after treatment were censored at Day 3. Also, results from the patient regular diary (by 15-day average) data were evaluated.

Data from 201 patients were used in the survival analysis. There were 101 patients in the mometasone furoate nasal spray group and 100 patients in the placebo group. From the individual patient onset diary data, it was found that there were a total of 24 patients who recorded slight or no relief (i.e. censored) at Day 3 in the mometasone furoate nasal spray group as compared to 50 patients in the placebo group similarly recording slight or no relief (i.e. censored).

Survival analysis results suggest that mometasone furoate nasal spray group had a median onset time to relief of 35.9 hours as compared to placebo group's 72 hours (due to more censored observations in this group). From a plot of the survival distribution for the two groups, it was seen that proportion reporting slight or no relief with increasing duration (in total hours) in the placebo group was higher compared to the mometasone furoate nasal spray group. Using a log-rank data showed a statistically significant difference between the two treatment groups (p-value <0.001).

Analysis of morning & evening averaged diary data showed that (for the 15-days average) reduction in the total nasal symptom score from baseline for mometasone furoate nasal spray group was statistically significantly higher than that for the placebo group.

In a first Phase I trial of the mometasone furoate dry powder inhaler (DPI), mometasone furoate-DPI was once-α-day given to eight normal volunteers in single doses of 400, 800, 1600, 3200 mcg and placebo. Parallel groups of volunteers received either budesondie dry powder (400, 800, 1600, 3200 mcg and placebo) or prednisone (5 mg, 10 mg, 20 mg, 40 mg, or placebo). All doses were administered at 11 p.m., and plasma cortisol levels over the next 24 hours were monitored.

### DRUG METABOLISM/CLINICAL PHARMACOLOGY STUDY

A drug metabolism and clinical pharmacology study was conducted by administering (by various routes) tritium-labeled mometasone furoate ("³H-MF") to 6 groups of 6 normal male volunteers in each group. Blood and urine samples were collected for measurement of total drug (including metabolites).

The objectives of these studies in male volunteers were to determine the absorption, metabolism and excretion of ³H-labeled mometasone furoate ("³H-MF") following administration by oral swallow as a solution and as an aqueous suspension of the monohydrate, by oral inhalation as a suspension from a standard metered dose inhaler (MDI) and from a metered dose inhaler containing a spacer device (Gentlehaler), by nasal inhalation as an aqueous suspension of the mometasone furoate monohydrate from a nasal spray unit and by intravenous injection as a solution.

### Population

Thirty-six (n=6 per treatment group) normal healthy male volunteers between the ages of 19 and 40 yr. (average 29 yr.) having weights in accordance with current actuarial tables (+ 10%) were enrolled in these single dose studies. All subjects were determined to be in good health by their medical history, physical examinations, clinical and laboratory tests.

### Study Design

Six volunteers in each of the six treatment groups received one of the following ³H-MF dosage forms listed in Table 1:

**TABLE 1**

| | Dose* | | |
|---|---|---|---|
| Dosage Form | mg/Subject | µCi/Subject | Mode of Administration |
| Oral Solution | 1.03 | 209 | 33.3 ml (0.031 mg/ml) by oral swallow |
| MDI (metered- dose inhaler) | 0.86 | 163 | 4 puffs from a MDI canister (215 µg/actuation) |
| Nasal Spray | 0.19 | 197 | 4 sprays from a nasal spray bottle (47 µg/spray) |
| Gentlehaler | 0.40 | 79 | 4 bursts from a MDI canister containing a spacer (referred to as Gentlehaler) (101 µg/burst) |
| Intravenous Solution | 1.03 | 204 | 1.03 mg/ml administered at a rate of 1 ml/min. |
| Oral Suspension (hydrated) | 0.99 | 195 | 1.6 ml (0.62 mg/ml by oral swallow |

| | | | |
|---|---|---|---|
| *Doses based on analysis of dosage forms prior to start of study | | | |

Plasma, urine, expired air filters, Respirgard and fecal samples were collected and assayed for radioactivity content. The limit of quantitation (LOQ) for plasma radioactivity ranged from 0.103 to 0.138 ng eq/ml., except for the nasal spray treatment where the LOQ was 0.025 ng eq/ml. Selected plasma, urine and fecal samples were analyzed for metabolite profiles.

### RESULTS

Clinical Summary - Mometasone furoate was found to be safe and well tolerated by all volunteers after administration of all dosage forms.

Pharmacokinetics - The mean (n=6) plasma concentrations of total radioactivity are illustrated in Summary Figure 1 and the mean (n=6) pharmacokinetic parameters derived from total plasma radioactivity are presented in Table 2.

Comparison of plasma radioactivity illustrated in Figure 1 and/or urinary excretion data and presented in Table 2 after the various formulations with those after intravenous treatment demonstrated that drug-derived radioactivity was completely absorbed when ³H-MF was administered orally as a solution. In contrast, systemic absorption of drug-derived radioactivity following administration of ³H-MF as an oral suspension or as a nasal spray suspension was approximately 8% of the dose. Systemic absorption of drug-derived radioactivity following administration of ³H-MF via the MDI (30%) and Gentlehaler™ (67%) was higher than that following nasal spray or oral suspension. Although the peak plasma concentration of radioactivity was less than 1 ng eq/ml for both MDI and Gentlehaler, comparative dose normalized AUC radioactivity data and urinary excretion data suggested that absorption of drug-derived radioactivity from the MDI and Gentlehaler was approximately 23-30% and 67-69%, respectively. The drug derived radioactivity data suggested that systemic bioavailability was greater following administration with the Gentlehaler™ compared to MDI administration. This may have been the result of enhanced lung deposition of drug due to the use of a spacer device in the Gentlehaler™. The Gentlehaler™ device is a MDI actuator described in USP 4,972,830.

Radioactivity was predominantly excreted in the feces regardless of dosage form and route of administration. Excretion of radioactivity in the urine was approximately 25% for the intravenous and oral solution formulations, 7% for the MDI and 16 % for the Gentlehaler and 2% or less for both the nasal spray and oral suspension formulations, respectively. These data thus demonstrate that the drug was well absorbed when orally administered as a solution formulation but poorly absorbed following oral or intranasal administration as a suspension formulation.

**TABLE 2**

| PHARMACOKINETIC PARAMETERS OF TOTAL RADIOACTIVITY FOLLOWING ADMINISTRATION OF ³H-MF IN MALE VOLUNTEERS | | | | | | |
|---|---|---|---|---|---|---|
| Dosage Form | | | | | | |
| | | | | | | |
| | | Oral | | | Nasal | Oral |
| Parameter | Intravenous | Solution | MDI | Gentlehaler | Spray | Suspension |
| Cmax | 23.7 | 4.8 | 0.80 (0.93*) | 0.69 (1.71*) | BQL** | BQL |
| AUC(1) | 401 | 488 | 81 (94*) | 110 (275') | BQL | BQL |
| Urine | 24 | 25 | 7 | 16 | 2 | 2 |
| (% dose) | | | | | | |
| Feces | 54 | 62 | 86 | 89 | 78 | 73 |
| (% dose) | | | | | | |
| U+F | 78 | 87 | 94 | 105 | 80 | 75 |
| (% dose) | | | | | | |
| % Absorbed | | | | | | |
| AUC | -- | 122 | 23* | 69* | -- | -- |
| Urine | -- | 104 | 30 | 67 | | 8 |

| | | | | | | |
|---|---|---|---|---|---|---|
| * Based on dose normalized data ** BQL = Below Quantifiable Limit | | | | | | |

| Parameter | Units | Definition |
|---|---|---|
| Cmax | ng eq/ml | Maximum plasma concentration, except for the intravenous treatment, |
| | | which is C₅ₘᵢₙ. |
| AUC(1) | ng eq hr/ml | Area under the plasma concentration-time curve to infinity. |
| Urine | | |
| (% dose) | % | Percent of administered radioactivity excreted in the urine through 168 hr. |
| Feces | | |
| (% dose) | % | Percent of administered radioactivity excreted in feces through 168 hr. |
| U+F | | |
| (% dose) | % | Total percent dose recovered in the urine and feces through 168 hr. |
| % Absorbed | | |
| (AUC) | % | Percent of administered radioactivity absorbed based on dose normalized |
| treatment data | | versus intravenous data. |
| % Absorbed | | |
| (Urine) | % | Percent of administered radioactivity absorbed (based on urinary excretion |
| data) | | compared to the intravenous dose. |

Selected plasma, urine and fecal extracts were analyzed by high performance liquid chromatography (HPLC) with radio-flow monitoring to determine metabolite profiles. The results of these analyses demonstrated that, following administration of the oral solution, most of the plasma radioactivity was associated with metabolites more polar than the available standards. Approximately 1.5% of the 3 hr. plasma radioactivity was associated with parent drug indicating extensive first past metabolism and rapid inactivation by the the liver. In contrast, following intravenous administration, approximately 39% of the 3 hr. plasma radioactivity was associated with parent drug. Approximately 12% and 33% of the 3 hr. plasma radioactivity was associated with parent drug following administration of the MDI and Gentlehaler, respectively. In general, the plasma concentrations of radioactivity following the nasal and oral suspension routes of administration were too low for metabolite profiling.

HPLC/radio-flow analysis of both urinary and fecal extracts following both intravenous and oral solution administration demonstrated that all of the radioactivity was associated with metabolites more polar than parent drug. Analysis of urine specimens obtained from subjects who received ³H-MF by the Gentlehaler also demonstrated that all of the radioactivity was associated with metabolites more polar than parent drug. However, analyses of fecal extracts following administration of the nasal spray, oral suspension and inhalation (MDI and Gentlehaler) formulations, demonstrated the presence primarily of mometasone furoate, presumably due to unabsorbed drug which was swallowed. Hydrolysis of plasma and urine was performed with an enzyme preparation containing both β-glucuronidase and aryl sulfatase. These experiments Yielded modest changes in the HPLC metabolite profiles that were consistent with the hydrolytic release of conjugated metabolites.

The percent of dose as tritiated water in the body was estimated from urinary distillation experiments to be approximately 3.7% after intravenous and 2.9% after oral solution dosing.

These findings suggested that less than 4% of the tritium label had exchanged with body water following administration of ³H-MF to male volunteers.

The results of these drug metabolism/clinical pharmacology studies demonstrate that:
1. Drug-derived radioactivity was completely absorbed when ³H-MF was given orally as a solution to male volunteers. However, the absolute bioavailability of unchanged mometasone furoate was extremely low (less than approximately 1 %) due to extensive first pass metabolism.
2. Drug-derived radioactivity was moderately absorbed following oral inhalation of ³H-MF by the metered dose inhaler (23-30%) and Gentlehaler™ (67-69%).
3. The absorption of drug-derived radioactivity following administration of ³H-MF nasal spray and oral suspension formulations was approximately 8%.
4. The plasma concentrations of unchanged mometasone furoate could not be determined after administration by oral inhalation as a suspension from a MDI or a Gentlehaler, or by nasal inhalation of an aqueous suspension of mometasone furoate monohydrate from a nasal spray unit or by oral swallow of an aqueous suspension of the monohydrate because of the plasma concentrations of total radioactivity were too low for metabolite profiling.
5. Mometasone furoate was extensively metabolized following all routes of administration.

As shown in Table 2, ³H-MF-derived radioactivity suggests that systemic absorption was greater from an orally swallowed solution (about 100%) than from an orally swallowed suspension or an intranasally inhaled suspension (8%). Mometasone furoate was detectable in plasma by metabolite profiling after administration of the drug by intravenous injection or oral administration as solution dosage forms, but not after administration of the oral or nasal suspensions. Similarly, the excretion of radioactivity in urine after dosing with the solution formulation was greater (25%) than after dosing with the nasal spray or oral suspension (2%). The total recovery or radioactivity in urine and feces was 87% and 75% respectively, with most of the radioactivity being excreted in the feces. After intravenous dosing, the total radioactivity excreted was 78% with 24% being excreted in the urine and 54% being excreted in the feces.

## Claims

1. An aqueous suspension of mometasone furoate for use as a medicament for the intranasal treatment of allergic rhinitis, by inserting an appropriate device into each nostril, wherein the medicament is formulated so that the amount of mometasone furoate administered is in the range of 200 to 400 mcg/day.

2. The aqueous suspension for the use of claim 1, wherein the medicament is formulated so that mometasone furoate is administered in single or divided doses.

3. The aqueous suspension for the use of claim 1 or claim 2, wherein the medicament is formulated so that the treatment is a once-a-day treatment.

4. The aqueous suspension for the use of claim 1 or claim 2, wherein the medicament is formulated so that mometasone furoate is administered for inhalation in dosages of 50, 100, 200 or 400 mcg 100 µL.

5. The aqueous suspension for the use of claim 3, wherein the medicament is formulated so that mometasone furoate is administered in dosages of 50 micrograms administered twice to each nostril once-a-day for a total once-a-day dose of 200 mcg.

## Patentansprüche

1. Eine wässrige Suspension von Mometasonfuroat zur Verwendung als ein Medikament zur intranasalen Behandlung von allergischer Rhinitis durch Einführen einer geeigneten Vorrichtung in jedes Nasenloch, wobei das Medikament so formuliert ist, dass die verabreichte Menge an Mometasonfuroat im Bereich von 200 bis 400 µg/Tag liegt.

2. Die wässrige Suspension zur Verwendung nach Anspruch 1, wobei das Medikament so formuliert ist, dass das Mometasonfuroat in einer Einzeldosis oder in Teildosen verabreicht wird.

3. Die wässrige Suspension zur Verwendung nach Anspruch 1 oder Anspruch 2, wobei das Medikament so formuliert ist, dass die Behandlung eine Einmal-pro-Tag-Behandlung ist.

4. Die wässrige Suspension zur Verwendung nach Anspruch 1 oder Anspruch 2, wobei das Medikament so formuliert ist, dass das Mometasonfuroat in Dosen von 50, 100, 200 oder 400 µg/100 µl zur Inhalation verabreicht wird.

5. Die wässrige Suspension zur Verwendung nach Anspruch 3, wobei das Medikament so formuliert ist, dass das Mometasonfuroat in Dosen von 50 Mikrogramm verabreicht wird, welche einmal pro Tag zweimal an jedes Nasenloch verabreicht werden, so dass eine Einmal-pro-Tag-Dosis von insgesamt 200 µg resultiert.

## Revendications

1. Suspension aqueuse de furoate de mométasone pour une utilisation en tant que médicament dans le traitement intranasal d'une rhinite allergique en introduisant un dispositif approprié dans chaque narine, le médicament étant formulé de sorte à ce que la quantité de furoate de mométasome est administrée à raison de 200 à 400 mcg/jour.

2. Suspension aqueuse pour l'utilisation selon la revendication 1, dans laquelle le médicament est formulé de sorte à ce que le furoate de mométasome est administré en une dose unique ou des doses fractionnées.

3. Suspension aqueuse pour l'utilisation selon la revendication 1 ou 2, dans laquelle le médicament est formulé de sorte à ce que le traitement est un traitement à une prise quotidienne unique.

4. Suspension aqueuse pour l'utilisation selon la revendication 1 ou 2, dans laquelle le médicament est formulé de sorte à ce que le furoate de mométasone est administré par une inhalation à des dosages de 50, 100, 200 ou 400 mcg/100µL.

5. Suspension aqueuse pour l'utilisation selon la revendication 3, dans laquelle le médicament est formulé de sorte à ce que le furoate de mométasone est administré dans des dosages de 50 microgrammes, administré deux fois dans chaque narine une fois par jour pour une dose totale quotidienne de 200 mcg.
